# EUROPEAN PATENT SPECIFICATION

(11) **EP 2 745 764 B1**
(45) Date of publication and mention of the grant of the patent: **18.07.2018**
(21) Application number: 13788143.9
(22) Date of filing: 08.04.2013
(51) Int. Cl.: A61B 1/00, A61B 8/12, A61B 10/02, A61B 17/28, A61B 10/04

(54) **TREATMENT INSTRUMENT FOR ENDOSCOPE**
ENDOSKOP-BEHANDLUNGSWERKZEUG
OUTIL DE TRAITEMENT DESTINÉ À UN ENDOSCOPE

(30) Priority: 10.05.2012 JP 2012108754
(43) Date of publication of application: 25.06.2014
(73) Proprietor: Olympus Corporation, Tokyo 192-8507 (JP)
(72) Inventor: NISHINA, Kenichi, Hachioji-shi, Tokyo 192-8507 (JP); BAN, Atsushi, Hachioji-shi, Tokyo 192-8507 (JP)
(74) Representative: Schicker, Silvia
(86) International application number: PCT/JP2013/060623
(87) International publication number: WO 2013/168498

(56) References cited:
- WO-A1-2010/071895
- WO-A1-2013/005500
- JP-A- H01 201 248
- JP-A- H08 299 346
- JP-A- 2002 306 606
- JP-U- H04 126 511
- US-A1- 2006 178 699
- US-A1- 2010 063 401
- US-A1- 2012 022 396
- US-B1- 6 299 622

## Description

### Technical Field

The present invention relates to a treatment instrument for an endoscope including a specimen sampling tool for sampling cells or tissues on an inner peripheral surface of a tubular subject.

### Background Art

A method is well known wherein after inserting an insertion portion of an endoscope to a vicinity of a small-diameter duct, into which the insertion portion of the endoscope cannot be inserted, such as a lung periphery of a bronchus, a bile duct, or a pancreatic duct in a subject, a tube provided with an X-ray marker on a distal end side is inserted into the small-diameter duct, to a vicinity of an examined region in the small-diameter duct under X-ray observation via a treatment instrument channel provided in the insertion portion, and, thereafter, a specimen sampling tool for sampling such as forceps or a puncture needle is inserted into the tube to sample cells or tissues of the examined region.

Cells or tissues of the examined region in a position opposed to a distal end of the tube in the duct can be sampled by the forceps or the puncture needle. However, since a space for changing a direction of the specimen sampling tool projecting from the tube distal end is absent in the small-diameter duct, there is a problem in that it is difficult to sample cells or tissues of the examined region located on an inner peripheral surface of the duct opposed to an outer peripheral surface of the tube.

Therefore, Japanese Patent Application Laid-Open Publication No. 2001-269345 discloses a specimen sampling tool provided with a brush at a distal end of a wire that can be inserted into a tube and can scrape off a cell on an inner peripheral surface of a duct.

However, the specimen sampling tool disclosed in Japanese Patent Application Laid-Open Publication No. 2001-269345 is configured to shave tissues of the subject surface by moving back and forth the brush pressed against the subject. This caused such problems that the specimen sampling tool cannot shave a large amount of tissues but the tissues shaved from the inner peripheral surface of the duct drop into the subject.

WO 2010/071895 A1, which is considered the closest prior art to the subject-matter of claim 1, discloses tools for accessing tissue for use with a locatable guide of a navigation system. Said tools are attachable to a distal tip of a locatable guide, such that the location of the tools is known while the tool is being used.

US 2012/022396 A1 discloses a device and method for collecting a tissue sample including a blade comprising an anterior portion and an elongated blade body. The elongated blade body is substantially firm and the anterior portion is bendable. The anterior portion comprises a sharp or pointed anterior end adapted to pierce and penetrate a tissue and a sampling mechanism with at least one recess or notch disposed therein or assembling channel to collect the tissue sample.

US 6,299,622 B1 discloses an atherectomy catheter for excising and imaging material in a body lumen. The catheter comprises a cutting blade which is configured to move between a first and second position relative to an aperture or cutting window.

US 2006/178699 A1 discloses a biopsy forceps including a plurality of grasping members extending from an inner shaft. The grasping members are biased toward an open configuration and constrained to a closed configuration by a sheath.

US 2010/063401 A1 discloses an ultrasound endoscope comprising a puncture needle which is located in a scan area of a first ultrasound image.

The present invention was made in view of these problems, and an objective of the present invention is to provide a treatment instrument for an endoscope which includes a specimen sampling tool capable of sampling more cells or tissues of an inner peripheral surface of a small-diameter duct than in conventional arts and which is configured to allow surely collecting cells or tissues sampled with the specimen sampling tool.

### Disclosure of Invention

### Means for Solving the Problem

The above captured problem is solved by a treatment instrument for an endoscope according to claim 1.

### Brief Description of the Drawings

Fig. 1 is a perspective view showing a treatment instrument for an endoscope in a first embodiment.
Fig. 2 is a partial sectional view of the treatment instrument for an endoscope taken along line II-II in Fig. 1.
Fig. 3 is a partial sectional view showing a state in which arm portions of a rod for cell sampling in a specimen sampling tool shown in Fig. 2 are further expanded in diameter than a tube.
Fig. 4 is a partial sectional view schematically showing a method of specifying an inclination angle of a through-hole of the tube shown in Fig. 2.
Fig. 5 is a partial perspective view showing in enlargement a distal end side of the arm portions shown in Fig. 2.
Fig. 6 is a partial perspective view showing a modification of a shape of a claw portion shown in Fig. 5.
Fig. 7 is a perspective view showing a modification of the treatment instrument for an endoscope shown in Fig. 1.
Fig. 8 is a partial sectional view of the treatment instrument for an endoscope taken along line VI-VI in Fig. 7.
Fig. 9 is a partial sectional view showing a state in which arm portions of a rod for cell sampling in a specimen sampling tool shown in Fig. 8 are further expanded in diameter than a tube.
Fig. 10 is a partial sectional view of a treatment instrument for an endoscope in a second embodiment taken along line VIII-VIII in Fig. 1.
Fig. 11 is a partial sectional view showing a state in which arm portions of a rod for cell sampling in a specimen sampling tool shown in Fig. 10 are further expanded in diameter than a tube.
Fig. 12 is a partial sectional view of the treatment instrument for an endoscope taken along line X-X in Fig. 7.
Fig. 13 is a partial sectional view showing a state in which the arm portions of the rod for cell sampling in the specimen sampling tool shown in Fig. 12 are further expanded in diameter than the tube.
Fig. 14 is a diagram schematically showing an endoscope system including the treatment instrument for an endoscope shown in Fig. 1.

### Best Mode for Carrying Out the Invention

Embodiments of the present invention are explained below with reference to the drawings.

### (First Embodiment)

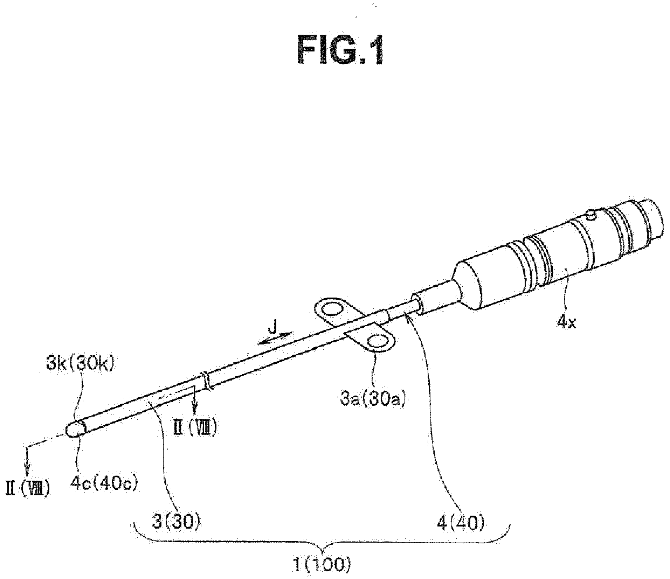

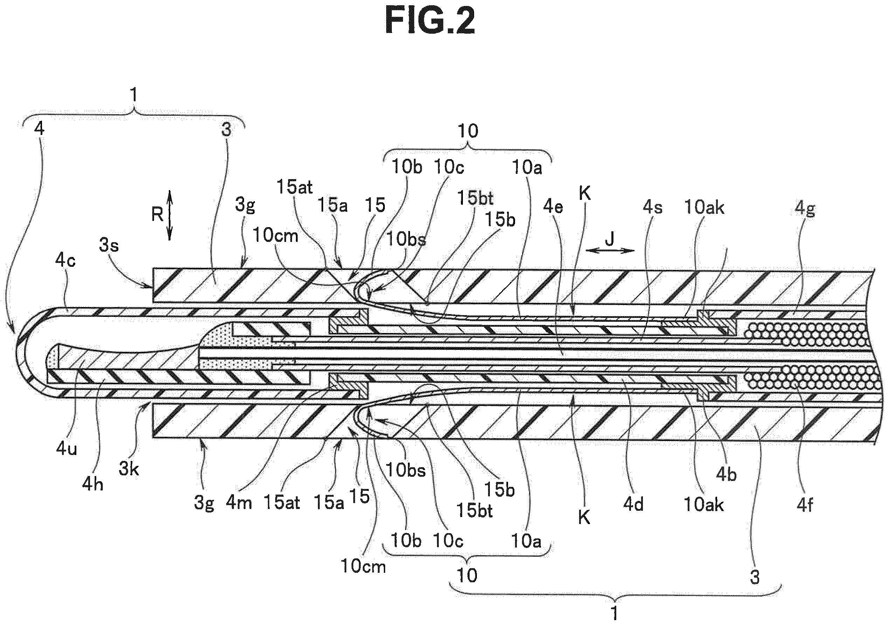

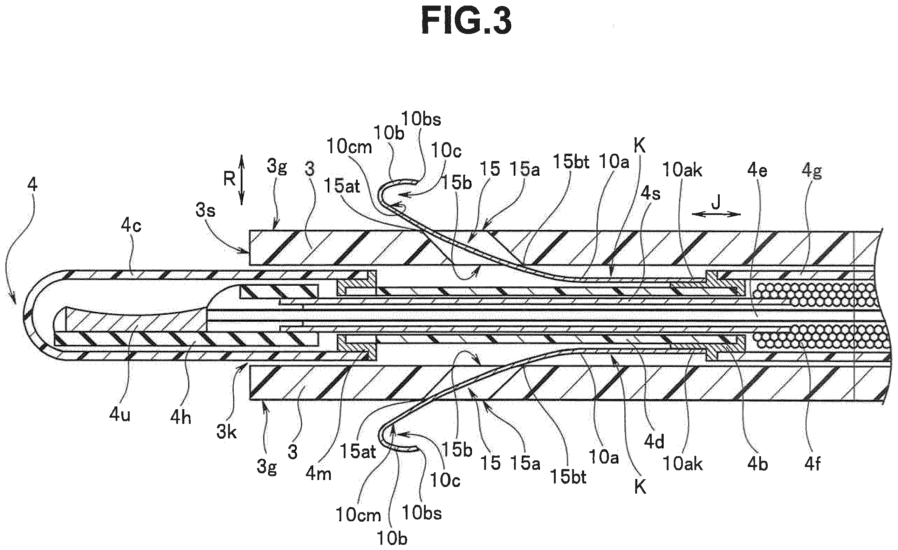

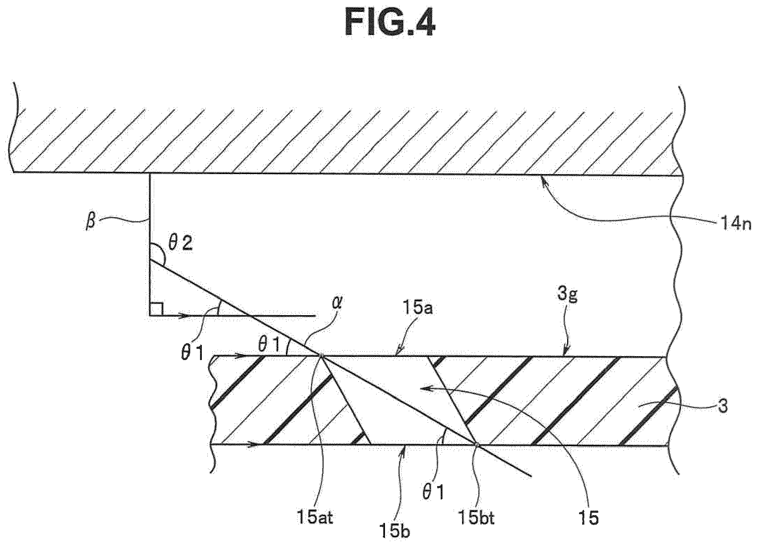

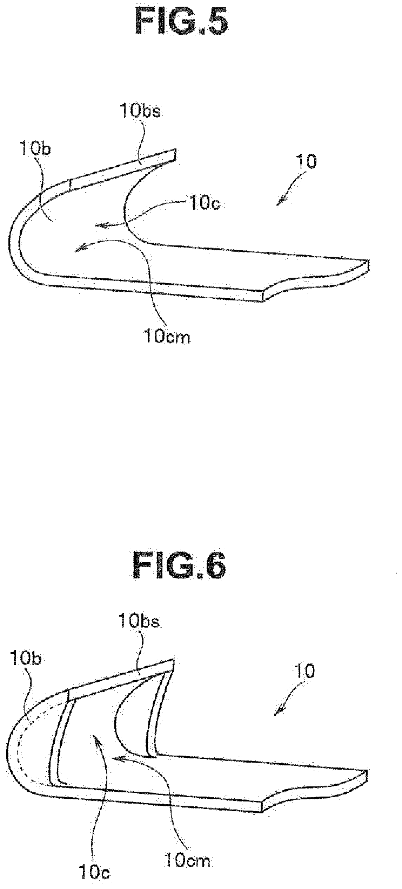

Fig. 1 is a perspective view showing a treatment instrument for an endoscope in a first embodiment. Fig. 2 is a partial sectional view of the treatment instrument for an endoscope taken along line II-II in Fig. 1. Fig. 3 is a partial sectional view showing a state in which arm portions of a rod for cell sampling in a specimen sampling tool shown in Fig. 2 are further expanded in diameter than a tube. Fig. 4 is a partial sectional view schematically showing a method of specifying an inclination angle of a through-hole of the tube shown in Fig. 2. Fig. 5 is a partial perspective view showing in enlargement a distal end side of the arm portions shown in Fig. 2. Fig. 6 is a partial perspective view showing a modification of a shape of a claw portion shown in Fig. 5.

As shown in Figs. 1 and 2, a main part of a treatment instrument for an endoscope 1 includes a tube 3 and a specimen sampling tool 4.

The tube 3 is formed to be elongated along an extending direction J of the specimen sampling tool 4. As shown in Figs. 1 and 2, the specimen sampling tool 4 is capable of moving back and forth in the extending direction J on an inside of the tube 3.

The tube 3 is inserted into a subject having a plurality of bent portions. Therefore, in order to secure insertability, the tube 3 is formed of resin having flexibility and having biocompatibility. Note that examples of the resin forming the tube 3 include polyethylene, fluororesin, and PEEK.

As shown in Fig. 1, on a proximal end side in the extending direction J of the tube 3, an operation portion 3a for rotating the tube 3 in a circumferential direction of the tube 3 and moving the tube 3 back and forth in the extending direction J is provided.

Note that, in the present embodiment, the specimen sampling tool 4 is configured with an ultrasound probe elongated along the extending direction J. More specifically, the specimen sampling tool 4 is configured with a mechanical radial scanning probe.

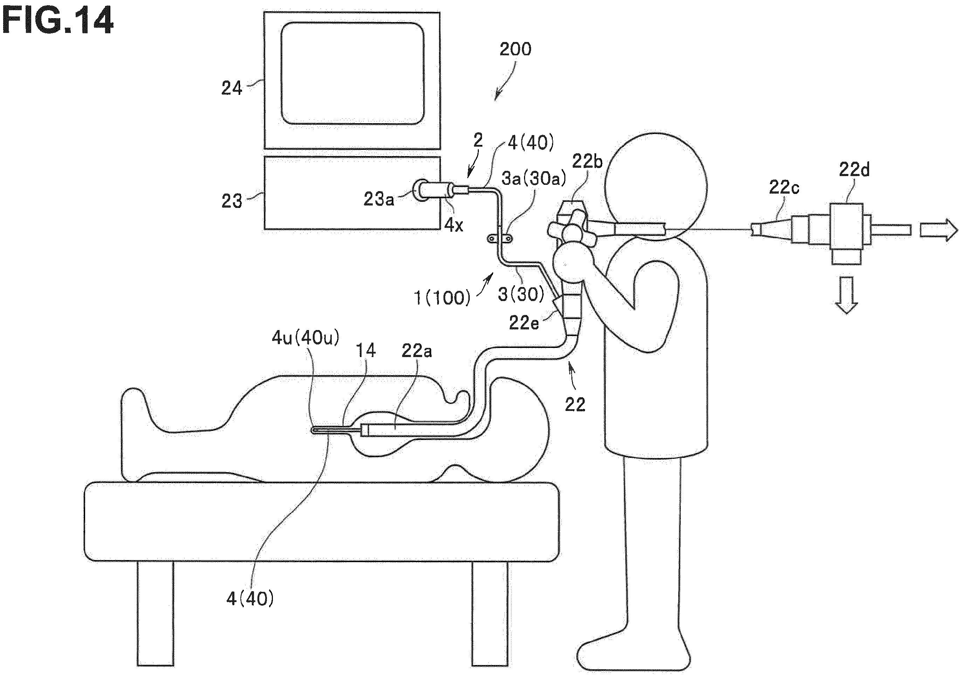

More specifically, the specimen sampling tool 4 includes an ultrasound observation portion 4u located at a distal end in the extending direction J and configured with a single ultrasound transducer of a single plate and a housing 4h configured to hold the ultrasound observation portion 4u. The specimen sampling tool 4 includes, toward a rear in the extending direction J from the ultrasound observation portion 4u, an ultrasound transducer cable 4e extended to a below-mentioned connector 4x (see Fig. 1) and configured to transmit an electric pulse signal from a below-mentioned ultrasound observation device 23 (see Fig. 14) to the ultrasound observation portion 4u.

The specimen sampling tool 4 includes a small-diameter shaft 4s including the ultrasound transducer cable 4e and extended backward in the extending direction J from the housing 4h. Further, the specimen sampling tool 4 includes a flexible shaft 4f including the ultrasound transducer cable 4e, fixed with a proximal end in the extending direction J of the small-diameter shaft 4s, and configured to apply rotation power to the housing 4h via the small-diameter shaft 4s.

Further, the specimen sampling tool 4 includes a sheath large-diameter portion 4g covering an outer periphery of the flexible shaft 4f, a cap 4b fixed to a distal end in the extending direction J of the sheath large-diameter portion 4g, and a sheath small-diameter portion 4d covering an outer periphery of the small-diameter shaft 4s and having a proximal end in the extending direction J fixed to the cap 4b. The specimen sampling tool 4 includes a cap 4m fixed to a proximal end in the extending direction J of the sheath small-diameter portion 4d and a distal end cap 4c covering the ultrasound observation portion 4u held by the housing 4h and having a distal end in the extending direction J fixed to the cap 4m. The specimen sampling tool 4 includes a rod for cell sampling 10.

That is, a main part of the specimen sampling tool 4 includes the ultrasound observation portion 4u, the housing 4h, the ultrasound transducer cable 4e, the small-diameter shaft 4s, the flexible shaft 4f, the sheath large-diameter portion 4g, the cap 4b, the sheath small-diameter portion 4d, the cap 4m, the distal end cap 4c, and the rod for cell sampling 10. The specimen sampling tool 4 is detachably attachable to the below-mentioned ultrasound observation device 23 (see Fig. 14) via the connector 4x provided at a proximal end in the extending direction J.

In the specimen sampling tool 4, according to relative movement with the tube 3 in the extending direction J, at least the ultrasound observation portion 4u is capable of projecting further forward in the extending direction J than the distal end 3s via an opening 3k formed at the distal end 3s in the extending direction J of the tube 3.

The sheath small-diameter portion 4d is formed in a small diameter further in a radial direction R of the specimen sampling tool 4 than the sheath large-diameter portion 4g and the distal end cap 4c, whereby, in a vicinity of an outer periphery of the sheath small-diameter portion 4d, a space K recessed in the radial direction R is formed between the cap 4m and the cap 4b along the extending direction J. The rod for cell sampling 10 is located in the space K.

More specifically, a main part of the rod for cell sampling 10 includes at least two arm portions 10a having flexibility and located along the extending direction J, claw portions 10b provided at distal ends in the extending direction J of the respective arm portions 10a, distal ends of the claw portions 10b shaving cells or tissues on an inner peripheral surface 14n (see Fig. 4) of a subject, and loading portions 10c provided in bent portions in the claw portions 10b, cells or tissues, which are collected specimens shaved by the claw portions 10b, being loaded on the loading portions 10c. In the rod for cell sampling 10, proximal ends 10ak in the extending direction J of the arm portions 10a are bound and fixed to the cap 4b by bonding, welding, brazing, or the like.

The rod for cell sampling 10 is configured of a material having predetermined hardness, having elasticity, and having biocompatibility, for example, a tabular member of a nickel titanium alloy, stainless steel for spring, or the like.

The claw portions 10b are formed to be bent to a loading surfaces 10cm side. As an example, the claw portions 10b are formed to be bent to an outer side in the radial direction R of the arm portions 10a such that an angle formed between the claw portions 10b and the arm portions 10a is equal to or smaller than 90°. Therefore, the claw portions 10b is formed in, for example, a hook shape.

More specifically, as shown in Fig. 5, the claw portion 10b is formed by bending a flat plate or, as shown in Fig. 6, the claw portion 10b may be formed in a box shape having sidewalls, for example, a shape like a basket of a digger by punching or the like. Further, the claw portion 10b may be formed integrally with the arm portion 10a or may be formed separately from the arm portion 10a via, for example, a link member.

The claw portion 10b is located in a linear through-hole 15 formed in an outer peripheral part on a distal end side in the extending direction J of the tube 3 and tilting such that an opening 15b on an inner side of the radial direction R is located further on a proximal end side in the extending direction J than an opening 15a on an outer side in the radial direction R. The claw portion 10b is capable of being retracted in the through-hole 15 from the outside of the tube 3.

Note that, as shown in Fig. 2, in a state in which the claw portions 10b are retracted in the through-hole 15, the distal end side in the extending direction J of the tube 3 is located to overlap the ultrasound observation portion 4u in the extending direction J.

The claw portions 10b are expanded further to an outer side in the radial direction R than an outer peripheral surface 3g of the tube 3 according to relative movement of the specimen sampling tool 4 and the tube 3 in the extending direction J, that is, whether the tube 3 is moved backward in the extending direction J with respect to the specimen sampling tool 4 or the specimen sampling tool 4 is moved forward in the extending direction J with respect to the tube 3.

More specifically, the bent portions of the claw portions 10b slip toward the opening 15a while coming into contact with a slope of the through-hole 15 with pressure and the arm portions 10a are pressed against an opening end 15bt on a proximal end side in the extending direction J of the opening 15b according to whether the tube 3 is moved backward in the extending direction J with respect to the specimen sampling tool 4 or the specimen sampling tool 4 is moved forward in the extending direction J with respect to the tube 3. Further, as shown in Fig. 3, the bent portions of the claw portions 10b and a distal end side in the extending direction J of the arm portions 10a are pressed against an opening end 15at on a distal end side in the extending direction J of the opening 15a, whereby the claw portions 10b are expanded in diameter to the distal end side in the extending direction J of the arm portions 10a and further to the outer side in the radial direction R than the outer peripheral surface 3g of the tube 3 from the through-hole 15.

That is, the distal end side in the extending direction J of the arm portions 10a and the claw portions 10b are physically expanded to the outer side in the radial direction R with the opening end 15at as a point of action by the slope of the through-hole 15 and the opening end 15at. Note that the diameter expansion of the claw portions 10b is recognized by an examiner under X-ray observation.

Therefore, the bent portions of the claw portions 10b come into contact with, with pressure, a part where the through-hole 15 is formed in the outer periphery of the tube 3. Therefore, it is desirable that the part is formed to be harder than other parts of the outer periphery of the tube 3 such that the part is not deformed by contact with the claw portions 10b and the claw portions 10b and the arm portions 10a can be physically expanded to the outer side in the radial direction R. For example, the part where the through-hole 15 is formed in the outer periphery of the tube 3 may be formed of metal.

As shown in Fig. 4, when an angle formed by a straight line α connecting the opening end 15bt on the proximal end side in the extending direction J of the opening 15b and the opening end 15at on the distal end side in the extending direction J of the opening 15a and the outer peripheral surface 3g of the tube 3 is represented as θ1 and an exterior angle in a crossing position of the straight line α and a straight line β perpendicular to the inner peripheral surface 14n of the subject and the outer peripheral surface 3g of the tube 3 is represented as θ2, the through-hole 15 is formed at an inclination angle satisfying 90° + θ1 ≥ θ2 > θ1.

This is because, if the through-hole 15 is formed at the inclination angle satisfying 90° + θ1 ≥ θ2 > θ1, for example, even if the claw portions 10b are bent or folded at an angle 90° formed between the claw portions 10b and the arm portions 10a, since sharp edges lObs of the distal ends of the claw portions 10b surely come into contact with the inner peripheral surface 14n (see Fig. 4) of the subject at an angle equal to or larger than 90°, cells or tissues on the inner peripheral surface 14n are easily shaved.

Note that, when the tube 3 is moved in the extending direction J relatively to the specimen sampling tool 4, as shown in Fig. 3, the ultrasound observation portion 4u completely projects further forward in the extending direction J than the opening 3k of the distal end 3s of the tube 3. Consequently, an observation of the examined region can be performed by the ultrasound observation portion 4u.

As shown in Fig. 3, in a state in which the claw portions 10b are expanded in diameter further to the outer side in the radial direction R than the outer peripheral surface 3g of the tube 3 from the through-hole 15 and the edges lObs at the distal ends of the claw portions 10b come into contact with the inner peripheral surface 14n of the subject at an angle equal to or larger than 90°, the specimen sampling tool 4 and the tube 3 are integrally repeatedly moved back and forth in the extending direction J under the observation by the ultrasound observation portion 4u, whereby the distal ends shave cells or tissues on the inner peripheral surface 14n. Note that the cells or the tissues shaved from the inner peripheral surface 14n by the edges lObs of the distal ends of the claw portions 10b are loaded on the loading surfaces 10cm of the loading portions 10c provided in the bent portions in the claw portions 10b.

After the diameter expansion of the claw portions 10b shown in Fig. 3, the claw portions 10b are retracted in the tube 3 according to relative movement of the specimen sampling tool 4 and the tube 3 opposite to the relative movement in the diameter expansion, more specifically, whether the tube 3 is moved forward in the extending direction J with respect to the specimen sampling tool 4 or the specimen sampling tool 4 is moved backward in the extending direction J with respect to the tube 3. More specifically, as shown in Fig. 2, the claw portions 10b are retracted in the through-hole 15 via the opening 15a. That is, the cells or the tissues loaded on the loading surfaces 10cm of the loading portions 10c are retracted in the through-hole 15.

Note that, as shown in Fig. 2, after the claw portions 10b are retracted in the through-hole 15, the distal end side in the extending direction J of the tube 3 is located to overlap the ultrasound observation portion 4u in the extending direction J. Therefore, since the distal end 3s of the tube 3 is observed by the ultrasound observation portion 4u, the examiner can easily recognize completion of the housing of the claw portions 10b in the through-hole 15 from a below-mentioned monitor 24 (see Fig. 14).

Conversely, when the distal end 3s of the tube 3 cannot be checked by the ultrasound observation portion 4u, the examiner can easily recognize from the monitor 24 (see Fig. 14) that the claw portions 10b have not been retracted in the through-hole 15 yet.

In a retracted state in the through-hole 15, most part of the opening 15a is closed by the bent portions of the claw portions 10b. Therefore, the cells or the tissues on the loading portions 10c less easily drop into the subject via the opening 15a.

Note that, after the retracting of the claw portions 10b in the through-hole 15, even if it is attempted to move the tube 3 forward in the extending direction J with respect to the specimen sampling tool 4 or move the specimen sampling tool 4 backward in the extending direction J with respect to the tube 3, the edges lObs of the distal ends of the claw portions 10b are caught by an inclined surface of the through-hole 15. Therefore, the claw portions 10b do not enter the tube 3.

Next, action in the present embodiment is briefly explained.

When it is desired to sample cells or tissues of a small-diameter duct 14 (see Fig. 14) into which an insertion portion 22a of an endoscope 22 (see Fig. 14 for both of the insertion portion 22a and the endoscope 22) cannot be inserted, for example, an examined region of a lung periphery, first, the insertion portion 22a of the endoscope 22 is inserted from a mouth of the subject into a bronchus of a lung as deep as possible.

Subsequently, the treatment instrument for an endoscope 1 inserted into a treatment instrument channel of the endoscope 22 is projected forward from a distal end of the treatment instrument channel and further inserted to a periphery of the bronchus. The treatment instrument for an endoscope 1 is inserted until a distal end in the extending direction J of the treatment instrument for an endoscope 1 is located in the vicinity of the examined region of the lung periphery under X-ray observation.

Note that, when it is difficult to insert the treatment instrument for an endoscope 1 into the lung periphery, it is also possible that, first, a guide smaller in diameter than the treatment instrument for an endoscope 1 is inserted into the lung periphery via the treatment instrument channel and, thereafter, the treatment instrument for an endoscope 1 is inserted into the lung periphery via the guide.

A position of the tube 3 of the treatment instrument for an endoscope 1 can be always recognized under X-ray observation.

When the vicinity of the distal end of the treatment instrument for an endoscope 1 reaches the vicinity of a lesion of the lung periphery, the ultrasound transducer 4u is driven to move the treatment instrument for an endoscope 1 back and forth and check a position of the lesion while performing an ultrasound observation.

Thereafter, the tube 3 is moved backward in the extending direction J with respect to, for example, the specimen sampling tool 4. As a result, as shown in Fig. 3, the ultrasound observation portion 4u completely projects forward in the extending direction from the opening 3k of the distal end 3s of the tube 3. At the same time, the claw portions 10b are expanded in diameter further to the outer side in the radial direction R than the outer peripheral surface 3g of the tube 3 from the through-hole 15 via the opening 15a. Note that the diameter expansion of the claw portions 10b is checked by the examiner under X-ray observation.

Subsequently, under an observation of the ultrasound observation portion 4u, the specimen sampling tool 4 and the tube 3 are moved forward or backward in the extending direction J. After, for example, the lesion is accurately grasped as an examined region of the lung periphery, in a state in which the edges 10bs of the distal ends of the claw portions 10b are in contact with the inner peripheral surface 14n, the specimen sampling tool 4 and the tube 3 are repeatedly moved back and forth in the extending direction J, whereby cells or tissues on the inner peripheral surface 14n are shaved by the edges 10bs of the distal ends of the claw portions 10b. At this point, the examined region is shaved by the claw portions 10b under the observation by the ultrasound observation portion 4u. Therefore, it is possible to surely shave cells or tissues in the examined region with high position accuracy without mistaking the examined region.

Note that the cells or the tissues shaved by the edges 10bs of the distal ends of the claw portions 10b are loaded on the loading surfaces 10cm of the loading portions 10c provided in the bent portion in the claw portions 10b. Thereafter, the tube 3 is moved forward in the extending direction J with respect to the specimen sampling tool 4, whereby the claw portions 10b are retracted in the through-hole 15 via the opening 15a. That is, the cells or the tissues loaded on the loading surfaces 10cm of the loading portions 10c are retracted in the through-hole 15.

Finally, the treatment instrument for an endoscope 1 is pulled out via the treatment instrument channel of the endoscope 22. The tube 3 is moved backward in the extending direction J with respect to the specimen sampling tool 4 again and the claw portions 10b are expanded in diameter further to the outer side in the radial direction R than the outer peripheral surface 3g of the tube 3 from the through-hole 15 via the opening 15a, whereby the cells or the tissues of the examined region loaded in the through-hole 15 can be easily collected.

Note that the above is the same when the small-diameter duct 14 is a bile duct or a pancreatic duct.

As explained above, in the present embodiment, it is explained that the specimen sampling tool 4 is inserted in the tube 3 to be capable of moving back and forth in the extending direction J. It is explained that the proximal ends of the arm portions 10a in the rod for cell samplings 10, in which the claw portions 10b that shave biological tissues are provided at the distal ends of the arm portions 10a and the loading portions 10c on which shaved biological tissues are loaded are provided in the bent portions of the claw portions 10b, is fixed to the specimen sampling tool 4. Further, it is explained that the claw portions 10b are capable of being expanded in diameter further to the outer side in the radial direction R than the outer peripheral surface 3g of the tube 3 and capable of being retracted in the through-hole 15 formed in the tube 3 according to relative movement of the tube 3 and the specimen sampling tool 4 in the extending direction J.

It is explained that, after the diameter expansion of the claw portions 10b, the edges lObs of the distal ends of the claw portions 10b come into contact with the inner peripheral surface 14n of the examined region at an angle equal to or larger than 90°.

Further, it is explained that, after the diameter expansion of the claw portions 10b, the tube 3 and the specimen sampling tool 4 are integrally moved back and forth in the extending direction in a state in which the edges lObs of the distal ends of the claw portions 10b are in contact with the inner peripheral surface 14n, whereby cells or tissues are shaved and the shaved cells or tissues are loaded on the loading surfaces 10cm of the loading portions 10c.

Consequently, the edges lObs of the distal ends of the claw portions 10b shave cells or tissues in a state in which the edges 10bs are in contact with the inner peripheral surface 14n of the examined region at an angle equal to or larger than 90°. As a result, the edges 10bs of the distal ends of the claw portions 10b can shave cells or tissues in a state in which the edges lObs are deeply stuck in the examined region. Therefore, it is possible to shave the cells or the tissues more than the conventional specimen sampling tool such as a brush.

The cells or the tissues shaved by the claw portions 10b are loaded on the loading surfaces 10cm of the loading portions 10c without dropping into the subject. After the claw portions 10b are retracted in the through-hole 15, the cells or the tissues are surely loaded in the through-hole 15 in a state in which the opening 15a of the through-hole 15 is closed by the bent portions of the claw portions 10b. Therefore, the cells or the tissues do not drop into the subject via the opening 15a.

When cells or tissues are shaved by the claw portions 10b, the shaving is performed under the observation by the ultrasound observation portion 4u of the specimen sampling tool 4. Therefore, it is possible to accurately sample cells or tissues of the examined region with high position accuracy.

A reason for the above is as explained below. Conventionally, under X-ray observation, first, the tube with the X-ray marker provided at the distal end in the extending direction is inserted into the small-diameter duct 14, in a state in which the distal end of the tube is located in the vicinity of the examined region, the ultrasound probe is inserted into the tube and an accurate position of the examined region is checked, and, thereafter, the ultrasound probe is pulled out from the tube, the specimen sampling tool such as the brush is inserted into the tube again, and sampling of cells or tissues is performed. However, under X-ray observation, a slight movement of the tube cannot be detected and, in addition, cells or tissues can not be sampled under the observation by the ultrasound probe. Therefore, after the ultrasound probe is pulled out from the tube, the position of the examined region deviates because of insertion work for the specimen sampling tool, pulmonary respiration, and the like in addition to this pull-out work. Therefore, it is likely that cells or tissues are sampled in a position greatly different from the examined region in the extending direction J. However, in this configuration, since cells or tissues can be sampled under the observation by the ultrasound probe, it is possible to accurately sample cells or tissues of the examined region.

Further, the replacement work for the ultrasound probe and the specimen sampling tool in the tube 3 in the past is unnecessary. Therefore, it is possible to simplify a manipulation for cell or tissue sampling.

Consequently, it is possible to provide the treatment instrument for an endoscope 1 including the specimen sampling tool 4 that can sample cells or tissues on the inner peripheral surface 14n in the small-diameter duct 14 more than in the past and including the configuration capable of surely collecting the cells or the tissues sampled by the specimen sampling tool 4 in the tube 3.

Certainty of a diagnosis of cells or tissues is improved. A sampling time for cells or tissues can be reduced. Further, it is unnecessary to perform re-examination in order to sample cells or tissues. Therefore, it is possible to reduce burdens on both of the examiner and the subject.

Note that in the present embodiment explained above, modifications of which are explained below, it is explained that the specimen sampling tool 4 is configured with the mechanical radial scanning probe. However, the specimen sampling tool 4 is not limited to this. Naturally, the specimen sampling tool 4 may be configured with a radial electronic scanning probe.

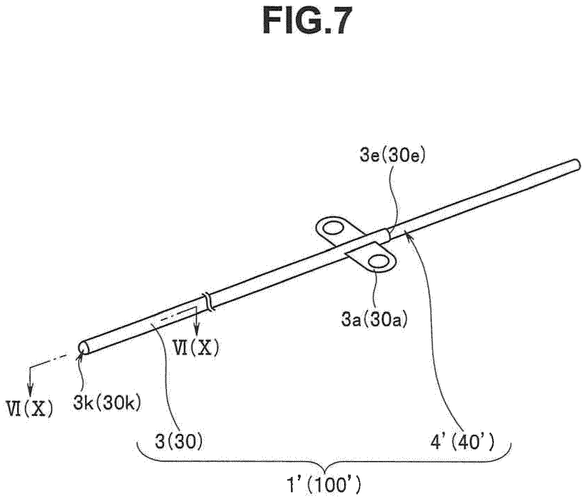

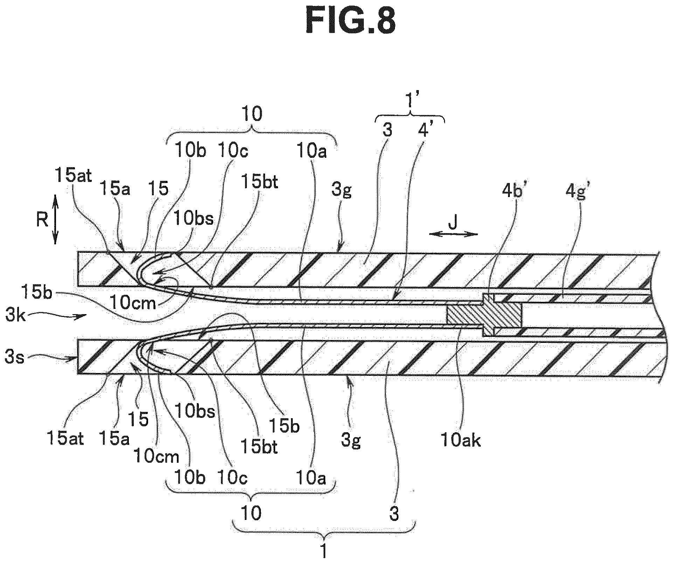

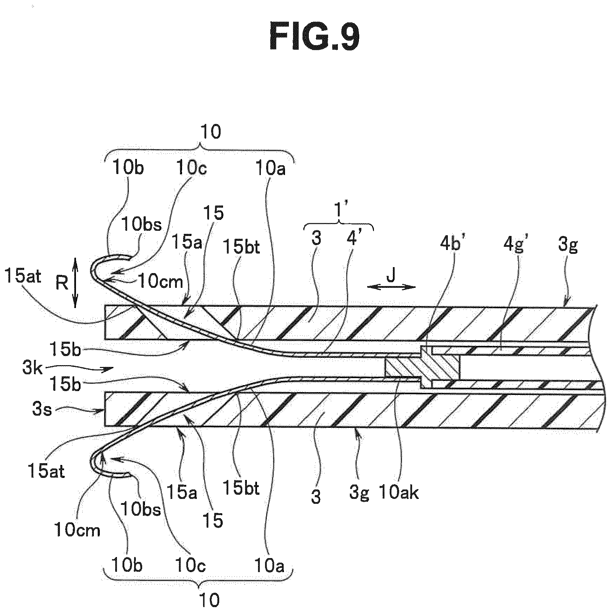

The modifications are explained below with reference to Figs. 7 to 9. Fig. 7 is a perspective view showing a modification of the treatment instrument for an endoscope shown in Fig. 1. Fig. 8 is a partial sectional view of the treatment instrument for an endoscope taken along line VI-VI in Fig. 7. Fig. 9 is a partial sectional view showing a state in which arm portions of a rod for cell sampling in a specimen sampling tool shown in Fig. 8 are further expanded in diameter than a tube.

In the present embodiment explained above, the specimen sampling tool 4 is explained with reference to the ultrasound probe as an example. However, the specimen sampling tool 4 is not limited to this. The specimen sampling tool may be a member elongated in the extending direction J including the rod for cell sampling 10 and having no observation function.

More specifically, as shown in Figs. 7 and 8, a main part of a treatment instrument for an endoscope 1' includes the tube 3 and a specimen sampling tool 4'.

As shown in Figs. 7 to 9, a main part of the specimen sampling tool 4' includes, in the tube 3, an inner tube 4g' extending further backward than a proximal end 3e in the extending direction J of the tube 3, a cap 4b', a proximal end side in the extending direction J of which is fixed to a distal end in the extending direction J of the inner tube 4g', and the rod for cell sampling 10, the proximal ends 10ak of the arm portions 10a of which are fixed to a distal end side in the extending direction J of the cap 4b', extending forward in the extending direction J from the cap 4b' in the tube 3.

Note that, also in the treatment instrument for an endoscope 1', according to relative movement in the extending direction J of the tube 3 and the specimen sampling tool 4, as shown in Fig. 9, at least the claw portions 10b are capable of being expanded in diameter further to the outer side in the radial direction R than the outer peripheral surface 3g of the tube 3 from the through-hole 15 and, as shown in Fig. 8, the claw portions 10b are capable of being retracted in the through-hole 15.

The other components and action other than observation performed using the ultrasound probe are the same as those in the present embodiment explained above.

With such a configuration of the treatment instrument for an endoscope 1', although cells or tissues cannot be sampled under an ultrasound observation, cells or tissues can be shaved more by the claw portions 10b than the conventional specimen sampling tool such as a brush as in the present embodiment explained above. Besides, the shaved cells or tissues can be surely collected by the loading portions 10c and the through-hole 15. Note that other effects are the same as those in the present embodiment explained above.

### (Second Embodiment)

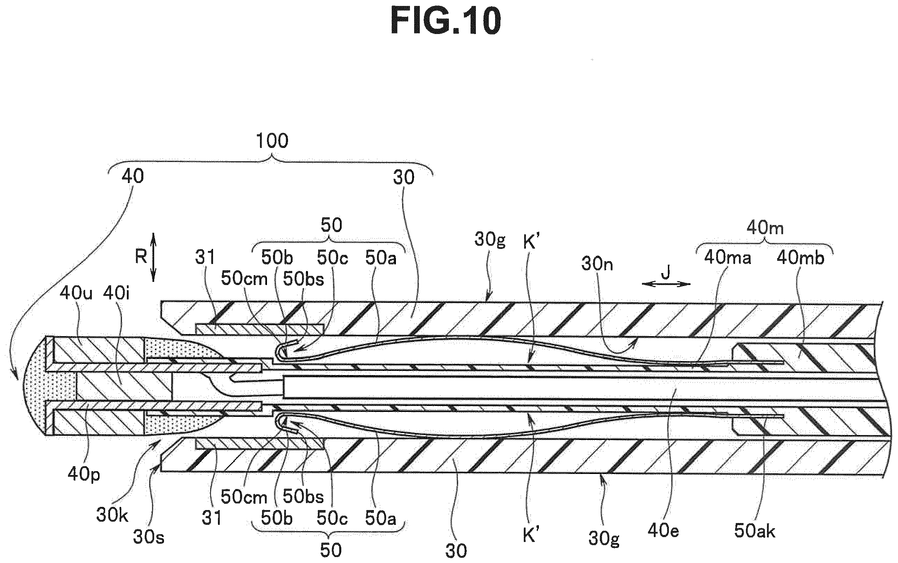

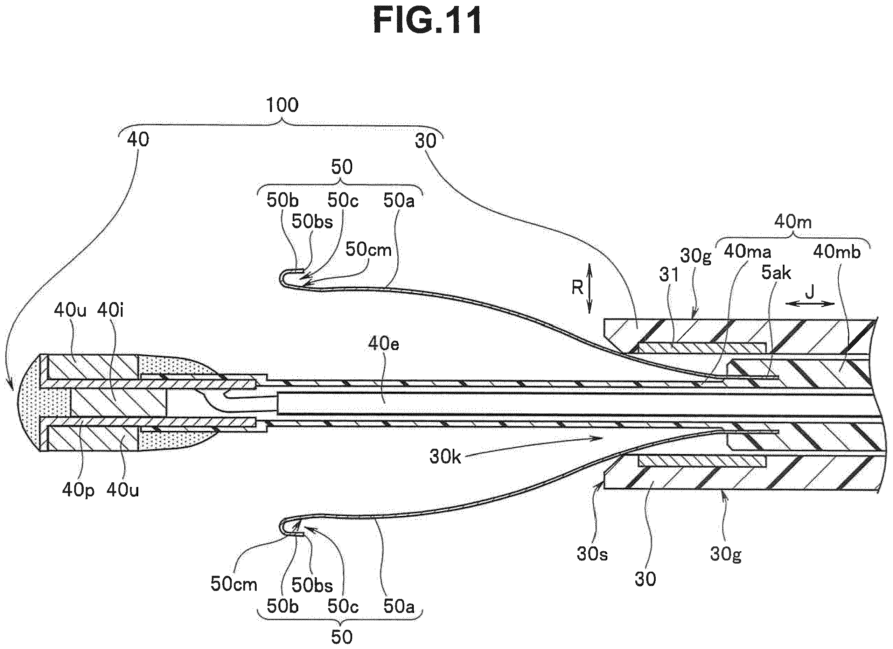

Fig. 10 is a partial sectional view of a treatment instrument for an endoscope in the present embodiment taken along line VIII-VIII in Fig. 1. Fig. 11 is a partial sectional view showing a state in which arm portions of a rod for cell sampling in a specimen sampling tool shown in Fig. 10 are further expanded in diameter than a tube.

Compared with the treatment instrument for an endoscope 1 in the first embodiment shown in Figs. 1 to 4, a configuration of the treatment instrument for an endoscope in the second embodiment is different in that a claw portion of the rod for cell sampling is expanded in diameter further to an outer side in a radial direction than an outer peripheral surface of the tube via an opening at a distal end of the tube according to relative movement of the tube and the specimen sampling tool. Therefore, components same as the components in the first embodiment are denoted by the same reference numerals and signs and explanation of the components is omitted.

As shown in Figs. 1 and 10, a main part of a treatment instrument for an endoscope 100 includes a tube 30 and a specimen sampling tool 40.

The tube 30 is formed to be elongated along the extending direction J of the specimen sampling tool 40. As shown in Figs. 1 and 10, the specimen sampling tool 40 is capable of moving back and forth in the extending direction J on an inside of the tube 30. Note that a material of the tube 30 is the same as the material of the tube 3.

As shown in Fig. 1, an operation portion 30a for rotating the tube 30 in a circumferential direction of the tube 30 and moving the tube 30 back and forth in the extending direction J is provided at the proximal end side in the extending direction of the tube 30.

Further, as shown in Fig. 10, a metal ring 31 is fixed to an inner peripheral surface on a distal end side in the extending direction J in an outer peripheral part of the tube 30 by press fitting or integral machining with the tube 30. A distal end 30s in the extending direction J of the tube 30 is chamfered.

In the present embodiment, the specimen sampling tool 40 is configured with an ultrasound probe elongated along the extending direction J.

The specimen sampling tool 40 is configured with a radial electronic scanning probe. More specifically, the specimen sampling tool 40 includes an ultrasound observation portion 40u located at a distal end in the extending direction J and configured with a ring-like ultrasound transducer and a cylindrical flexible substrate 40p, on an outer peripheral surface on a distal end side in the extending direction J of which the ultrasound observation portion 40u is mounted. Further, the specimen sampling tool 40 includes an integrated circuit element 40i mounted on an inner peripheral surface of the flexible substrate 40p and configured to process ultrasound signals transmitted and received by the ultrasound observation portion 40u and an ultrasound transducer cable 40e, a distal end in the extending direction J of which is electrically connected to the flexible substrate 40p, extended to the connector 4x (see Fig. 1) and configured to transmit an electric pulse signal received from the below-mentioned ultrasound observation device 23 (see Fig. 14) to the ultrasound observation portion 40u.

The specimen sampling tool 40 includes a probe insertion portion 40m that covers an outer circumference of the ultrasound transducer cable 4e and a distal end in the extending direction of which is fixed to an outer peripheral surface on a proximal end side in the extending direction of the flexible substrate 40p.

A main part of the probe insertion portion 40m includes a large-diameter portion 40mb and a small-diameter portion 40ma located further on a distal end side in the extending direction J than the large-diameter portion 40mb and configured to cover a distal end side in the extending direction J of the ultrasound transducer cable 40e. The specimen sampling tool 40 includes a rod for cell sampling 50.

That is, a main part of the specimen sampling tool 40 includes the ultrasound observation portion 40u, the flexible substrate 40p, the integrated circuit element 40i, the ultrasound transducer cable 40e, the probe insertion portion 40m, and the rod for cell sampling 50. The specimen sampling tool 40 is detachably attachable to the below-mentioned ultrasound observation device 23 (see Fig. 14) via the connector 4x provided at a proximal end in the extending direction J.

In the specimen sampling tool 40, according to relative movement of the tube 30 and the specimen sampling tool 40 in the extending direction J, at least the ultrasound observation portion 40u is capable of projecting further forward in the extending direction J than the distal end 30s via an opening 30k formed at the distal end 30s in the extending direction J of the tube 30.

A main part of the rod for cell sampling 50 includes at least two arm portions 50a having flexibility and located along the extending direction J, claw portions 50b provided at distal ends in the extending direction J of the respective arm portions 50a, distal ends of the claw portions 50b shaving cells or tissues on the inner peripheral surface 14n (see Fig. 4) of a tubular subject, and loading portions 50c provided in bent portions in the claw portions 50b, cells or tissues, which are collected specimens shaved by the claw portions 50b, being loaded on the loading portions 50c. In the rod for cell sampling 50, proximal ends 50ak in the extending direction J of the arm portions 50a are bound and fixed to a vicinity of a boundary between the large-diameter portion 40mb and the small-diameter portion 40ma of the probe insertion portion 40m by any one of bonding, insertion, and the like.

The arm portions 50a of the rod for cell sampling 50 is configured with a member having elasticity, for example, stainless steel for spring, a nickel titanium alloy, a cobalt chrome alloy, or the like. Further, as shown in Fig. 11, in a natural state, the arm portions 50a is formed such that a diameter in the radial direction R between the at least two claw portions 50b is larger than a diameter in the radial direction R of the tube 30. The arm portions 50a are reduced in diameter against the elasticity in the radial direction R and retracted in the tube 30.

In the arm portions 50a, as shown in Fig. 10, in a state in which the arm portions 50a are reduced in diameter and retracted in the tube 30, the claw portions 50b and a proximal end side in the extending direction J of the arm portions 50a are in non-contact with an inner peripheral surface 30n in an outer peripheral part of the tube 30. The other parts are retracted while having a convex shape on an outer side in the radial direction R that is in contact with the inner peripheral surface 30n.

Note that, in the retracted state, the rod for cell sampling 50 is located in a space K', which is formed because the small-diameter portion 40ma is smaller in diameter in the radial direction R than the large-diameter portion 40mb, along a vicinity of an outer periphery of the small-diameter portion 40ma.

Note that configurations and action of the claw portions 50b and the loading portions 50c are the same as those of the claw portions 10b and the loading portions 10c in the first embodiment. Therefore, explanation of the configurations and the action is omitted.

In the present embodiment, the claw portions 50b are capable of being retracted in the tube 30 from an outside of the tube 30 via the opening 30k of the distal end 30s in the extending direction J of the tube 30.

The claw portions 50b are expanded in diameter further to the outer side in the radial direction R than an outer peripheral surface 30g of the tube 30 according to relative movement of the specimen sampling tool 40 and the tube 30 in the extending direction J, that is, whether the tube 30 is moved backward in the extending direction J with respect to the specimen sampling tool 40 or the specimen sampling tool 40 is moved forward in the extending direction J with respect to the tube 30.

More specifically, the claw portions 50b together with a distal end side in the extending direction J of the arm portions 50a are expanded in diameter further to the outer side in the radial direction R than the outer peripheral surface 30g of the tube 30 by an elastic restoration force of the arm portions 50a via the opening 30k according to whether the tube 30 is moved backward in the extending direction J with respect to the specimen sampling tool 40 or the specimen sampling tool 40 is moved forward in the extending direction J with respect to the tube 30. Note that the diameter expansion of the claw portions 50b is checked by an examiner under X-ray observation.

When the tube 30 is moved in the extending direction J relatively to the specimen sampling tool 40, as shown in Fig. 11, the ultrasound observation portion 40u completely projects further forward in the extending direction J than the opening 30k of the distal end 30s of the tube 30. Consequently, the observation of the examined region can be performed by the ultrasound observation portion 40u.

As shown in Fig. 11, in a state in which the claw portions 50b are expanded in diameter further to the outer side in the radial direction R than the outer peripheral surface 30g of the tube 30 from the opening 30k and edges 50bs at the distal ends of the claw portions 50b come into contact with the inner peripheral surface 14n of the subject at an angle equal to or larger than 90°, the specimen sampling tool 40 and the tube 30 are integrally repeatedly moved back and forth in the extending direction J under the observation by the ultrasound observation portion 40u, whereby the distal ends shave cells or tissues on the inner peripheral surface 14n. Note that the cells or the tissues shaved from the inner peripheral surface 14n by the edges 50bs of the distal ends of the claw portions 50b are retracted on the loading surfaces 50cm of the loading portions 50c provided in the bent portions in the claw portions 50b.

After the diameter expansion of the claw portions 50b shown in Fig. 11, the claw portions 50b are reduced in diameter and retracted in the tube 30 via the opening 30k as shown in Fig. 10 according to relative movement of the specimen sampling tool 40 and the tube 30 opposite to the relative movement in the diameter expansion, more specifically, whether the tube 30 is moved forward in the extending direction J with respect to the specimen sampling tool 40 or the specimen sampling tool 40 is moved backward in the extending direction J with respect to the tube 30.

At this point, since the distal end 30s of the tube 30 is chamfered and a taper surface is formed at the distal end 30s, the claw portions 50b are not caught by the distal end 30s. The claw portions 50b are guided by the taper surface and smoothly retracted in the tube 30.

As explained above, the arm portions 50a has a shape in which the claw portions 50b and the proximal end side in the extending direction J of the arm portions 50a are non-contact with the inner peripheral surface 30n in the outer peripheral part of the tube 30 and the other parts are retracted while having the convex shape on the outer side in the radial direction R that is in contact with the inner peripheral surface 30n. Consequently, as shown in Fig. 10, after the claw portions 50b are retracted in the tube 30, when the rod for cell sampling 50 is retracted in the tube 30 via the opening 30k, only the proximal end side in the extending direction J of the arm portions 50a comes into contact with the distal end 30s of the tube 30, that is, the claw portions 50b do not come into contact with the distal end 30s of the tube 30. Therefore, the sampled cells or tissues are not caught by the distal end 30s of the tube 30.

In this retracted state, most part of the opening 30k is closed by the ultrasound observation portion 40u. Therefore, the cells or the tissues on the loading portions 50c less easily drop into the subject via the opening 30k.

Note that, after the retracting of the claw portions 50b in the tube 30, it is possible to easily collect the cells or the tissues by pulling out the specimen sampling tool 40 from the tube 30.

When the treatment instrument for an endoscope 100 has such a configuration, effects same as the effects in the first embodiment can be obtained. Further, since the specimen sampling tool 40 can be removed in a state in which the tube 30 is stored in the subject, it is possible to repeatedly perform cell sampling. It is possible to insert a specimen sampling tool such as a brush, a puncture needle, or forceps into the tube 30 instead of the specimen sampling tool 40 to perform sampling of cells or tissues. It is possible to easily perform a treatment manipulation such as radio wave cauterization following the sampling of cells or tissues.

Note that, in the present embodiment explained above, modifications of which are explained below, it is explained that the specimen sampling tool 40 is configured with the radial electronic scanning probe. However, the specimen sampling tool 40 is not limited to this. Naturally, the specimen sampling tool 40 may be configured with a mechanical radial scanning probe.

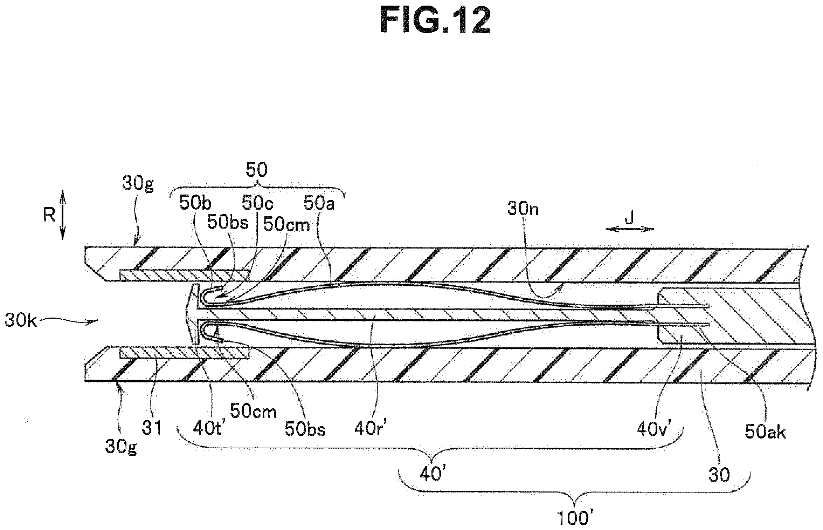

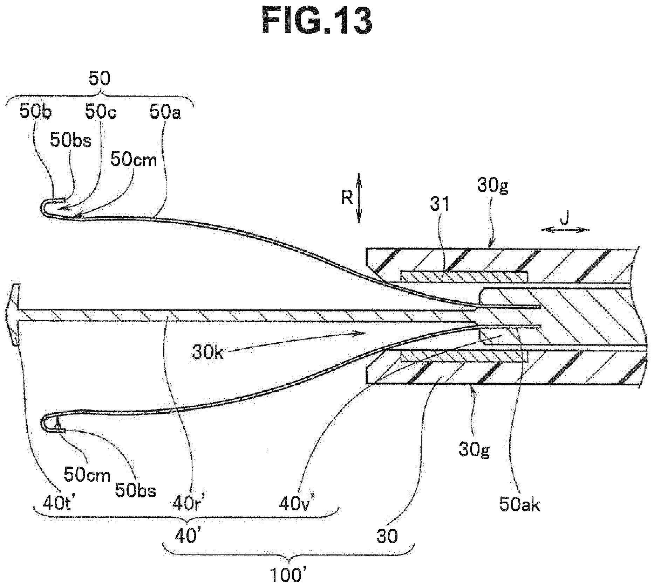

The modifications are explained below with reference to Figs. 12 and 13. Fig. 12 is a partial sectional view of the treatment instrument for an endoscope taken along line X-X in Fig. 7. Fig. 13 is a partial sectional view showing a state in which the arm portions of the rod for cell sampling in the specimen sampling tool shown in Fig. 12 is further expanded in diameter than the tube.

In the present embodiment explained above, it is explained that the specimen sampling tool 40 is the ultrasound probe. However, the specimen sampling tool 40 is not limited to this. The specimen sampling tool may be a member elongated in the extending direction J including the rod for cell sampling and having no observation function.

More specifically, as shown in Figs. 12 and 13, a main part of a treatment instrument for an endoscope 100' includes the tube 30 and a specimen sampling tool 40'.

As shown in Figs. 12 and 13, a main part of the specimen sampling tool 40' includes, in the tube 30, a probe insertion portion large-diameter portion 40v' extending further backward than a proximal end 30e in the extending direction J of the tube 30, a probe insertion portion small-diameter portion 40r' projecting forward in the extending direction J from the probe insertion portion large-diameter portion 40v', a brim portion 40t' provided at a distal end in the extending direction J of the probe insertion portion small-diameter portion 40r', and the rod for cell sampling 50.

Note that, the proximal ends 50ak of the arm portions 50a of the rod for cell sampling 50 are fixed to a vicinity of a boundary between the probe insertion portion large-diameter portion 40v' and the probe insertion portion small-diameter portion 40r' by any one of bonding, insertion, and the like. Therefore, in the rod for cell sampling 50, the claw portions 50b are located further on the proximal end side in the extending direction J than brim portions 50t and the arm portions 50a are located along an outer periphery of the probe insertion portion small-diameter portion 40r'.

Note that, also in the treatment instrument for an endoscope 100', according to relative movement in the extending direction J of the tube 30 and the specimen sampling tool 40', as shown in Fig. 13, the claw portions 50b together with the distal end side in the extending direction J of the arm portions 50a are capable of being expanded in diameter further to the outer side in the radial direction R than the outer peripheral surface 30g of the tube 30 from the opening 30k and, as shown in Fig. 12, the claw portions 50b are capable of being retracted in the tube 30 via the opening 30k.

After the sampling of the cells or the tissues, as in the present embodiment explained above, the cells or the tissues loaded on the loading surfaces 50cm of the loading portions 50c are retracted in the tube 30. At this point, the brim portion 40t' prevents the cells or the tissues from dropping into the subject via the opening 30k. Therefore, the brim portion 40t' only has to be provided according to necessity and is not an essential component.

The other components and action other than observation performed using the ultrasound probe are the same as those in the present embodiment explained above.

With such a configuration of the treatment instrument for an endoscope 100', although cells or tissues cannot be sampled under an ultrasound observation, cells or tissues can be shaved more by the claw portions 50b than the conventional specimen sampling tool such as a brush as in the present embodiment explained above. Further, the shaved cells or tissues can be surely collected by the loading portions 50c and the opening 30k. Note that, other effects are the same as those in the present embodiment explained above.

In the examples explained in the first and second embodiments explained above, the number of the arm portions 10a and 50a of the rods for cell sampling 10 and 50 fixed to the specimen sampling tools 4, 4', 40, and 40' is two. However, the number of the arm portions 10a and 50a is not limited to this. It goes without saying that the arm portions 10a and 50a may be provided in any number.

As the number of the arm portions 10a and 50a is larger, it is less necessary to perform alignment for rotating the specimen sampling tools 4, 4', 40, and 40' in the circumferential direction with respect to the examined region and bringing the claw portions 10b and 50b into contact with the examined region. That is, it is easy to align the claw portions 10b and 50b with the examined region.

Note that the treatment instrument for an endoscope 1 in the first embodiment and the treatment instrument for an endoscope 100 in the second embodiment explained above are used for, for example, an endoscope system shown in Fig. 14.

Fig. 14 is a diagram schematically showing an endoscope system including the treatment instrument for an endoscope shown in Fig. 1.

As shown in Fig. 14, the endoscope 22 included in an endoscope system 200 includes the elongated endoscope insertion portion 22a having flexibility. An operation portion 22b is provided on an examiner side of the endoscope insertion portion 22a.

Further, a universal cord 22c is extended from the operation portion 22b. A scope connector 22d is provided at an end of the universal cord 22c. A video processor device and a light source device not shown in the figure are connected to the scope connector 22d.

A treatment instrument insertion port 22e is opened in the vicinity of a coupling portion of the endoscope insertion portion 22a and the operation portion 22b. The treatment instrument channel is inserted through the treatment instrument insertion port 22e. The treatment instrument channel is formed in the endoscope insertion portion 22a. A distal end of the treatment instrument channel is opened to a distal end face of the endoscope insertion portion 22a. The connector 4x provided at the proximal end of the specimen sampling tool 4 (40) of the treatment instrument for an endoscope 1 (100) is connected to a connector receiving portion 23a of the ultrasound observation device 23. The ultrasound observation device 23 causes the monitor 24 to display an ultrasound image obtained by the ultrasound observation portion 4u (40u).

## Claims

1. A treatment instrument for an endoscope (1) comprising:
a specimen sampling tool (4) including at least two arm portions (10a) having flexibility, and loading portions (10c) provided at a distal end in an extending direction (J) of the arm portions (10a), a collected specimen from a subject being loaded on the loading portion (10c), and claw portions (10b) provided at the distal end of the arm portions (10a), a proximal end in the extending direction (J) of the arm portions (10a) being bound and fixed to the specimen sampling tool (4); and
a tube (3) on an inside of which the specimen sampling tool (4) is placed to be capable of moving back and forth in the extending direction (J), wherein
at least the claw portions (10b) of the arm portions (10a) are expanded in diameter toward an outer side of the tube (3) in a radial direction (R), further than an outer peripheral surface (3g) of the tube (3) according to relative movement of the specimen sampling tool (4) and the tube (3) in the extending direction (J), and
after the diameter expansion, the arm portions (10a) are retracted in the tube according to relative movement of the specimen sampling tool and the tube opposite to the relative movement in the diameter expansion in the extending direction (J),
**characterized in that** the claw portions (10b) are formed by a flat plate which is bent to a loading surface side (10cm) of the loading portion (10c), on which the collected specimen is loaded, to include the loading portion on an inside, and the claw portions being configured to shave the subject.

2. The treatment instrument for an endoscope (1) according to claim 1, wherein
a through-hole (15) is formed in an outer periphery part (3g) on a distal end side in the extending direction (J) of the tube (3),
at least the claw portions (10b) of the arm portions (10a) are expanded in diameter further to an outer side of the tube (3) in the radial direction (R) than the tube (3) via the through-hole (15) according to the relative movement of the specimen sampling tool (4) and the tube (3) in the extending direction (J), and
after the diameter expansion, the arm portions (10a) are retracted in the through-hole (15) according to the relative movement of the specimen sampling tool (4) and the tube (3) opposite to the relative movement in the diameter expansion in the extending direction (J).

3. The treatment instrument for an endoscope (1) according to claim 2, wherein
the through-hole (15) is formed to tilt such that an opening(15b) on an inner side in the radial direction (R) is located further on a proximal end side in the extending direction (J) than an opening (15a) on an outer side in the radial direction (R), and
at least the claw portions (10b) of the arm portions (10a) are expanded in diameter to the outer side in the radial direction (R) by being pressed against an end of the opening (15a) on the outer side of the through-hole (15) from the through-hole (15) according to the relative movement of the specimen sampling tool (4) and the tube (3) in the extending direction (J).

4. The treatment instrument for an endoscope (1) according to claim 2, wherein a part of the tube (3) where the through-hole (15) is formed to be harder than other parts of the tube (3).

5. The treatment instrument for an endoscope (1) according to claim 1, wherein
the arm portions (10a) are configured of an elastic member, in which a diameter in the radial direction (R) between at least two of the claw portions (10b) after the diameter expansion is formed larger than a diameter of the tube (3), and are reduced in diameter against elasticity and stored in the tube (3),
at least the claw portions (10b) of the arm portions (10a) are expanded in diameter further to the outer side of the tube (3) in the radial direction (R) than the tube (3) by an elastic restoration force of the arm portions (10a) via an opening (3k) at a distal end (3s) in the extending direction (J) of the tube (3) according to the relative movement of the specimen sampling tool (4) and the tube (3) in the extending direction (J), and
after the diameter expansion, the diameter of the arm portions (10a) are reduced and retracted in the tube (3) via the opening (3k) according to the relative movement of the specimen sampling tool (4) and the tube (3) opposite to the relative movement in the diameter expansion in the extending direction (J).

6. The treatment instrument for an endoscope (1) according to claim 5, wherein, the arm portions (10a) are in a diameter reduced state in the tube (3), the claw portions (10b) and a proximal end of the arm portions (10a) are in non-contact with an inner peripheral surface of the tube (3) and other parts of the arm portions (10a) have a convex shape on an outer side in the radial direction (R) that is in contact with the inner peripheral surface.

7. The treatment instrument for an endoscope (1) according to claim 1, wherein
a distal end in the extending direction (J) of the tube (3) is opened, and
the specimen sampling tool (4) is an ultrasound probe in which at least a part of an ultrasound observation portion (4u) is projected further forward in the extending direction (J) than the distal end of the tube (3) via the opening (3k).

8. The treatment instrument for an endoscope (1) according to claim 7, wherein, when at least the claw portions (10b) are expanded in diameter further to an outer side of the tube (3) in the radial direction (R) than the tube (3), the ultrasound observation portion (4u) completely projects further forward in the extending direction (J) than the opening (3k) of the tube (3).

9. The treatment instrument for an endoscope (1) according to claim 7, wherein a distal end side (3s) in the extending direction (J) of the tube (3) is located to overlap the ultrasound observation portion (4u) in the extending direction (J) when the arm portions (10a) are retracted in the tube (3).

## Patentansprüche

1. Behandlungsinstrument für ein Endoskop (1), umfassend:
ein Probenentnahmewerkzeug (4), das mindestens zwei Flexibilität aufweisende Armabschnitte (10a) und Ladeabschnitte (10c), die an einem distalen Ende in einer Ausfahrrichtung (J) der Armabschnitte (10a) vorgesehen sind, eine aus einem Subjekt entnommene Probe, die auf den Ladeabschnitt (10c) geladen ist, und Greifabschnitte (10b), die an dem distalen Ende der Armabschnitte (10a) vorgesehen sind, umfasst, ein proximales Ende in der Ausfahrrichtung (J) der Armabschnitte (10a), das gebogen und an dem Probenentnahmewerkzeug (4) fixiert ist; und
ein Rohr (3), an dessen Innenseite das Probenentnahmewerkzeug (4) platziert ist, um in der Ausfahrrichtung (J) vor- und zurückbewegt werden zu können, wobei
mindestens die Greifabschnitte (10b) der Armabschnitte (10a) im Durchmesser in Richtung einer Außenseite des Rohrs (3) in einer Radialrichtung (R) weiter aufgeweitet sind als eine Außenumfangsfläche (3g) des Rohrs (3) entsprechend einer relativen Bewegung des Probenentnahmewerkzeugs (4) und des Rohrs (3) in der Ausfahrrichtung (J), und
nach der Durchmesseraufweitung die Armabschnitte (10a) in das Rohr zurückgefahren werden entsprechend einer relativen Bewegung des Probenentnahmewerkzeugs und des Rohrs entgegengesetzt zu der relativen Bewegung bei der Durchmesseraufweitung in der Ausfahrrichtung (J),
**dadurch gekennzeichnet, dass** die Greifabschnitte (10b) durch eine ebene Platte gebildet sind, die auf eine Ladeoberflächenseite (10cm) des Ladeabschnitts (10c), auf dem die entnommene Probe geladen ist, gebogen wird, um den Ladeabschnitt auf einer Innenseite zu enthalten, und wobei die Greifabschnitte dazu ausgelegt sind, das Subjekt zu schaben.

2. Behandlungssystem für ein Endoskop (1) nach Anspruch 1, wobei
ein Durchgangsloch (15) in einem Außenumfangsteil (3g) auf einer Distalendseite in der Ausfahrrichtung (J) des Rohrs (3) gebildet ist,
mindestens die Greifabschnitte (10b) der Armabschnitte (10a) an einer Außenseite des Rohrs (3) in der Radialrichtung (R) über das Durchgangsloch (15) im Durchmesser weiter aufgeweitet werden als das Rohr (3) entsprechend der relativen Bewegung des Probenentnahmewerkzeugs (4) und des Rohrs (3) in der Ausfahrrichtung (J), und
nach der Durchmesseraufweitung die Armabschnitte (10a) in das Durchgangsloch (15) zurückgefahren werden entsprechend der relativen Bewegung des Probenentnahmewerkzeugs (4) und des Rohrs (3) entgegengesetzt zu der relativen Bewegung bei der Durchmesseraufweitung in der Ausfahrrichtung (J).

3. Behandlungsinstrument für ein Endoskop (1) nach Anspruch 2, wobei
das Durchgangsloch (15) dazu gebildet ist, sich derart zu neigen, dass sich eine Öffnung (15b) auf einer Innenseite in der Radialrichtung (R) weiter an einer Proximalendseite in der Ausfahrrichtung (J) befindet als eine Öffnung (15a) auf einer Außenseite in der Radialrichtung (R), und
mindestens die Greifabschnitte (10b) der Armabschnitte (10a) zur Außenseite in der Radialrichtung (R) im Durchmesser aufgeweitet werden, indem sie gegen ein Ende der Öffnung (15a) an der Außenseite des Durchgangslochs (15) gedrückt werden, von dem Durchgangsloch (15) entsprechend der relativen Bewegung des Probenentnahmewerkzeugs (4) und des Rohrs (3) in der Ausfahrrichtung (J).

4. Behandlungsinstrument für ein Endoskop (1) nach Anspruch 2, wobei ein Teil des Rohrs (3), dort wo das Durchgangsloch (15) gebildet ist, härter gebildet ist, als andere Teile des Rohrs (3).

5. Behandlungssystem für ein Endoskop (1) nach Anspruch 1, wobei
die Armabschnitte (10a) aus einem elastischen Element ausgelegt sind, in dem ein Durchmesser in der Radialrichtung (R) zwischen mindestens zwei der Greifabschnitte (10b) nach der Durchmesserausdehnung größer gebildet ist als ein Durchmesser des Rohrs (3), und im Durchmesser gegen Elastizität reduziert und in dem Rohr (3) gelagert sind,
mindestens die Greifabschnitte (10b) der Armabschnitte (10a) zur Außenseite des Rohrs (3) in der Radialrichtung (R) im Durchmesser weiter ausgedehnt werden als das Rohr (3) durch eine elastische Wiederherstellungskraft der Armabschnitte (10a) über eine Öffnung (3k) an einem distalen Ende (3s) in der Ausfahrrichtung (J) des Rohrs (3) gemäß der relativen Bewegung des Probenentnahmewerkzeugs (4) und des Rohrs (3) in der Ausfahrrichtung (J), und
nach der Durchmesseraufweitung die Durchmesser der Armabschnitte (10a) reduziert und in das Rohr (3) zurückgefahren werden über die Öffnung (3k) gemäß der relativen Bewegung des Probenentnahmewerkzeugs (4) und des Rohrs (3) entgegengesetzt zu der relativen Bewegung in der Durchmesseraufweitung in der Ausfahrrichtung (J).

6. Behandlungsinstrument für ein Endoskop (1) nach Anspruch 5, wobei, die Armabschnitte (10a) befinden sich in einem durchmesserreduzierten Zustand in dem Rohr (3), die Greifabschnitte (10b) und ein proximales Ende der Armabschnitte (10a) in Nichtkontakt mit einer Innenumfangsfläche des Rohrs (3) sind und andere Teile der Armabschnitte (10a) eine konvexe Form an einer Außenseite in der Radialrichtung (R) aufweisen, die in Kontakt mit der Innenumfangsfläche ist.

7. Behandlungssystem für ein Endoskop (1) nach Anspruch 1, wobei
ein distales Ende in der Ausfahrrichtung (J) des Rohrs (3) offen ist, und
das Probenentnahmewerkzeug (4) eine Ultraschallsonde ist, in der mindestens ein Teil eines Ultraschallbeobachtungsabschnitts (4u) in der Ausfahrrichtung (J) weiter vorgerückt wird als das distale Ende des Rohrs (3) über die Öffnung (3k).

8. Behandlungsinstrument für ein Endoskop (1) nach Anspruch 7, wobei, wenn mindestens die Greifabschnitte (10b) im Durchmesser zu einer Außenseite des Rohrs (3) in der Radialrichtung (R) weiter aufgeweitet werden als das Rohr (3), der Ultraschallbeobachtungsabschnitt (4u) in der Ausfahrrichtung (J) vollständig weiter vorwärtsrückt als die Öffnung (3k) des Rohrs (3).

9. Behandlungsinstrument für ein Endoskop (1) nach Anspruch 7, wobei eine Distalendseite (3s) in der Ausfahrrichtung (J) des Rohrs (3) positioniert ist, um den Ultraschallbeobachtungsabschnitt (4u) in der Ausfahrrichtung (J) zu überlagern, wenn die Armabschnitte (10a) in das Rohr (3) zurückgefahren werden.

## Revendications

1. Instrument de traitement pour un endoscope (1) comprenant :
un outil d'échantillonnage de spécimen (4) comprenant au moins deux parties de bras (10a) ayant une certaine souplesse, et des parties de chargement (10c) disposées au niveau d'une extrémité distale dans une direction d'extension (J) des parties de bras (10a), un spécimen prélevé chez un sujet étant chargé sur la partie de chargement (10c), et des parties de griffe (10b) disposées au niveau de l'extrémité distale des parties de bras (10a), une extrémité proximale dans la direction d'extension (J) des parties de bras (10a) étant liée et fixée à l'outil d'échantillonnage de spécimen (4) ; et
un tube (3) à l'intérieur duquel l'outil d'échantillonnage de spécimen (4) est placé pour être capable de se déplacer selon un mouvement de va-et-vient dans la direction d'extension (J),
au moins les parties de griffe (10b) des parties de bras (10a) étant étendues en diamètre vers un côté externe du tube (3) dans une direction radiale (R), davantage qu'une surface périphérique externe (3g) du tube (3) selon un déplacement relatif de l'outil d'échantillonnage de spécimen (4) et du tube (3) dans la direction d'extension (J), et
après l'extension de diamètre, les parties de bras (10a) étant rétractées dans le tube selon un déplacement relatif de l'outil d'échantillonnage de spécimen et du tube opposé au déplacement relatif dans l'extension de diamètre dans la direction d'extension (J),
**caractérisé par le fait que** les parties de griffe (10b) sont formées par une plaque plate qui est courbée vers un côté de surface de chargement (10cm) de la partie de chargement (10c), sur laquelle le spécimen prélevé est chargé, pour inclure la partie de chargement à l'intérieur, et les parties de griffe étant configurées pour raser le sujet.

2. Instrument de traitement pour un endoscope (1) selon la revendication 1, dans lequel
un trou traversant (15) est formé dans une partie périphérique externe (3g) sur un côté d'extrémité distale dans la direction d'extension (J) du tube (3),
au moins les parties de griffe (10b) des parties de bras (10a) sont étendues en diamètre davantage vers un côté externe du tube (3) dans la direction radiale (R) que le tube (3) par l'intermédiaire du trou traversant (15) selon le déplacement relatif de l'outil d'échantillonnage de spécimen (4) et du tube (3) dans la direction d'extension (J), et
après l'extension de diamètre, les parties de bras (10a) étant rétractées dans le trou traversant (15) selon le déplacement relatif de l'outil d'échantillonnage de spécimen (4) et du tube (3) opposé au déplacement relatif dans l'extension de diamètre dans la direction d'extension (J).

3. Instrument de traitement pour un endoscope (1) selon la revendication 2, dans lequel
le trou traversant (15) est formé de façon à s'incliner de telle sorte qu'une ouverture (15b) sur un côté interne dans la direction radiale (R) est située davantage sur un côté d'extrémité proximale dans la direction d'extension (J) qu'une ouverture (15a) sur un côté externe dans la direction radiale (R), et
au moins les parties de griffe (10b) des parties de bras (10a) sont étendues en diamètre vers le côté externe dans la direction radiale (R) en étant pressées contre une extrémité de l'ouverture (15a) sur le côté externe du trou traversant (15) à partir du trou traversant (15) selon le déplacement relatif de l'outil d'échantillonnage de spécimen (4) et du tube (3) dans la direction d'extension (J).

4. Instrument de traitement pour un endoscope (1) selon la revendication 2, dans lequel une partie du tube (3) où se trouve le trou traversant (15) est formée de façon à être plus dure que d'autres parties du tube (3).

5. Instrument de traitement pour un endoscope (1) selon la revendication 1, dans lequel
les parties de bras (10a) sont configurées d'un élément élastique, dans lequel un diamètre dans la direction radiale (R) entre au moins deux des parties de griffe (10b) après l'extension de diamètre est formé de façon à être plus grand qu'un diamètre du tube (3), et ont un diamètre réduit par rapport à l'élasticité et sont stockées dans le tube (3),
au moins les parties de griffe (10b) des parties de bras (10a) sont étendues en diamètre davantage vers le côté externe du tube (3) dans la direction radiale (R) que le tube (3) par une force de restauration élastique des parties de bras (10a) par l'intermédiaire d'une ouverture (3k) au niveau d'un extrémité distale (3s) dans la direction d'extension (J) du tube (3) selon le déplacement relatif de l'outil d'échantillonnage de spécimen (4) et du tube (3) dans la direction d'extension (J), et
après l'extension de diamètre, le diamètre des parties de bras (10a) est réduit et rétracté dans le tube (3) par l'intermédiaire de l'ouverture (3k) selon le déplacement relatif de l'outil d'échantillonnage de spécimen (4) et du tube (3) opposé au déplacement relatif dans l'extension de diamètre dans la direction d'extension (J).

6. Instrument de traitement pour un endoscope (1) selon la revendication 5, dans lequel les parties de bras (10a) sont dans un état de diamètre réduit dans le tube (3), les parties de griffe (10b) et une extrémité proximale des parties de bras (10a) ne sont pas en contact avec une surface périphérique interne du tube (3) et d'autres parties des parties de bras (10a) ont une forme convexe sur un côté externe dans la direction radiale (R) qui est en contact avec la surface périphérique interne.

7. Instrument de traitement pour un endoscope (1) selon la revendication 1, dans lequel
une extrémité distale dans la direction d'extension (J) du tube (3) est ouverte, et
l'outil d'échantillonnage de spécimen (4) est une sonde ultrasonore dans laquelle au moins une partie d'une partie d'observation d'ultrasons (4u) est projetée davantage vers l'avant dans la direction d'extension (J) que l'extrémité distale du tube (3) par l'intermédiaire de l'ouverture (3k).

8. Instrument de traitement pour un endoscope (1) selon la revendication 7, dans lequel, lorsqu'au moins les parties de griffe (10b) sont étendues en diamètre davantage vers un côté externe du tube (3) dans la direction radiale (R) que le tube (3), la partie d'observation d'ultrasons (4u) se projette complètement davantage vers l'avant dans la direction d'extension (J) que l'ouverture (3k) du tube (3).

9. Instrument de traitement pour un endoscope (1) selon la revendication 7, dans lequel un côté d'extrémité distale (3s) dans la direction d'extension (J) du tube (3) est situé de façon à chevaucher la partie d'observation d'ultrasons (4u) dans la direction d'extension (J) lorsque les parties de bras (10a) sont rétractées dans le tube (3).
